# EUROPEAN PATENT SPECIFICATION

(11) **EP 0 556 241 B1**
(45) Date of publication and mention of the grant of the patent: **10.01.1996**
(21) Application number: 91919487.8
(22) Date of filing: 08.11.1991
(51) Int. Cl.: A61K 39/04

(54) **MYCOBACTERIUM VACCAE IN THE TREATMENT OF UVEITIS**
MYCOBACTERIUM VACCAE IN DER BEHANDLUNG VON UVEITIS
UTILISATION DE MYCOBACTERIUM VACCAE POUR LE TRAITEMENT DE L'UVEITE

(30) Priority: 08.11.1990 GB 9024320
(43) Date of publication of application: 25.08.1993
(73) Proprietor: UNIVERSITY COLLEGE LONDON, London WC1E 6HA (GB)
(72) Inventor: ROOK, Graham Arthur William, Haver Hill, Suffolk CB9 7EJ (GB); STANFORD, John Lawson, Claygate, Marden, Kent TN12 9TE (GB)
(74) Representative: Collier, Jeremy Austin Grey
(86) International application number: GB9101970
(87) International publication number: WO9208484

(56) References cited:
- EP-A- 0 262 710
- WO-A-85/03639
- WO-A-85/05034
- WO-A-91/02542
- Proceedings of the National Academy of Sciences, volume 85, June 1988,
- Biochemistry (Washington DC,US) D. Young et al.: "Stress proteins are immune targets in leprosy and tuberculosis", pages 4267-4270, see the whole article (cited in the application)

## Description

This invention relates to the treatment of uveitis.

British Specification No. 2156673 and International Publication WO 85/03639 describes immunotherapeutic agents comprising killed cells of Mycobacterium vaccae. These agents are useful in the immunotherapy of mycobacterial disease, especially tuberculosis and leprosy. It is stated that use of this immunotherapeutic agent facilitates the removal of the persisting bacilli responsible for tuberculosis or leprosy which, as is well known, it is difficult to remove by chemotherapy alone. It is suggested in the specification that the immunotherapeutic agent is believed to act by presenting the "protective" common mycobacterial antigens to advantage and by containing immune suppressor determinants which are active in regulating disadvantageous immune mechanisms. As a consequence, "persister" bacilli are recognized by the immune system by their content of common mycobacterial antigens and effective immune mechanisms are directed against them, in the absence of the tissue necrotic form of immunity usually present in mycobacterial disease.

International Patent Specification PCT/GB 85/00183 Publication No. WO 85/05034 describes compositions for the alleviation of the symptoms of, and for the treatment or diagnosis of, arthritic diseases which comprise as active ingredient the whole organism of M. vaccae. It is stated that the preparations of M. vaccae are useful for the treatment of various autoimmune diseases and especially arthritic conditions including rheumatoid arthritis, ankylosing spondylitis or Reiter's syndrome.

Uveitis is a condition, often observed in leprosy patients but also found in other individuals, which is difficult to treat and leads to permanent blindness. The present invention is founded upon the surprising observation that compositions comprising antigenic and immunoregulatory material derived from Mycobacterium vaccae are useful in the treatment of uveitis.

A method for the treatment of uveitis comprises administering to the patient suffering from such a condition an effective amount of a therapeutic composition comprising antigenic and immunoregulatory material derived from Mycobacterium vaccae.

The invention provides antigenic and immunoregulatory material derived from M. vaccae for use in the manufacture of a therapeutic agent for the treatment of uveitis. Such antigenic and immunoregulatory material is also provided for use in the manufacture of a therapeutic agent for use in the treatment of uveitis.

The therapeutic agent of the invention conveniently, and therefore preferably, comprises dead cells of M. vaccae, most preferably cells which have been killed by autoclaving or by irradiation. The therapeutic agent normally comprises more than 10⁸ microorganisms per ml of diluent, and preferably from 10⁸ to 10¹¹ killed M. vaccae microorganisms per ml of diluent.

The diluent may be pyrogen-free saline for injection alone, or a borate buffer of pH 8.0. The diluent should be sterile. A suitable borate buffer is:

| | |
|---|---|
| Na₂B₄0₇.10H₂0 | 3.63 g |
| H₃BO₃ | 5.25 g |
| NaC1 | 6.19 g |
| Tween® 80 | 0.0005% |
| Distilled Water | to 1 litre |

The preferred strain of M. vaccae is one denoted R877R isolated from mud samples from the Lango district of Central Uganda (J.L. Stanford and R.C. Paul, Ann. Soc. Belge Med, Trop. 1973, 53 141-389). The strain is a stable rough variant and belongs to the aurum sub-species. It can be identified as belonging to M. vaccae by biochemical and antigenic criteria (R. Bonicke, S.E. Juhasz., Zentr albl. Bakteriol. Parasitenkd. Infection skr. Hyg. Abt. 1, Orig., 1964, 192, 133).

The strain denoted R877R has been deposited under the Budapest Convention at the National Collection of Type Cultures (NCTC) Central Public Health Laboratory, Colindale Avenue, London NW9 5HT, United Kingdom on February 13th, 1984 under the number NCTC 11659.

For the preparation of the therapeutic agent, the microorganism M. vaccae may be grown on a suitable solid medium. A modified Sauton's liquid medium is preferred (S.V. Boyden and E. Sorkin., J. Immunol, 1955 75, 15) solidified with agar. Preferably the solid medium contains 1.3% agar. The medium inoculated with the microorganisms is incubated aerobically to enable growth of the microoganisms to take place, generally at 32°C for 10 days. The organisms are harvested, then weighed and suspended in a diluent. The diluent may be unbuffered saline but is preferably borate-buffered and contains a surfactant such as Tween® 80 as described above. The suspension is diluted to give 100 mg of microorganism/ml. For further dilution, borate buffered saline is preferably used so that the suspension contains 10 mg wet weight of microorganisms/ml of diluent. The suspension may then be dispensed into 5 ml multidose vials. Although the microorganisms in the vials may be killed using irradiation e.g. from ⁶⁰Cobalt at a dose of 2.5 megarads, or by any other means, for example chemically, it is preferred to kill the microorganisms by autoclaving, for example at 10 psi (69 kPa) for 10 minutes (115°-125°C). It has been discovered, unexpectedly, that autoclaving yields a more effective preparation than irradiation.

The therapeutic agent is in general administered by injection in a volume in the range 0.1-0.2 ml, preferably 0.1 ml, given intradermally. A single dosage will generally contain from 10⁷ to 10¹⁰ killed M. vaccae microorganisms. It is preferred to administer to patients a single dose containing 10⁸ to 10⁹ killed M. vaccae. However, the dose may be repeated depending on the condition of the patient.

While the present invention does not depend on the truth of this theory it is believed that the active ingredient in the killed M. vaccae may be the 65 kDa mycobacterial heat shock protein (hsp 65) described by Young et al. "Stress proteins are immune targets in leprosy and tuberculosis", Proc. Natl. Acad. Sci. U.S.A. 85 (1988), pp4267-4270 in a form obtained from M. bovis. The preferred autoclaved M. vaccae cells used in the present invention are believed to provide an effective package of the hsp 65 and other substances in a convenient adjuvant.

Although the therapeutic agent will generally be administered by intradermal injection, other routes, e.g. oral administration, can also be used.

It may be advantageous and is within the scope of the invention to use more than one strain of M. vaccae, and/or to include in the immunoprophylactic agent other mycobacterial antigens. Tuberculin may also be included.

The immunoprophylactic agent may also contain BCG (Bacillus Calmette-Guerin) vaccine, in particular the freeze-dried form of the vaccine, to promote its effect.

The therapeutic agent can contain further ingredients such as adjuvants, preservatives, stabilisers etc. It may be supplied in sterile injectable liquid form or in sterile freeze-dried form which is reconstituted prior to use.

M. vaccae may be used as such or as an extract or fractioned portion of the organism to manufacture the therapeutic agents according to the invention.

The following Example illustrates the invention.

### EXAMPLE

M. vaccae NCTC 11659 is grown on a solid medium comprising modified Sauton's medium solidified with 1.3% agar. The medium is inoculated with the microorganism and incubated for 10 days at 32°C to enable growth of the microorganism to take place. The microorganisms are then harvested by gently scraping the surface of the agar and weighed (without drying) and suspended in M/15 borate buffered saline at pH8 to give 10 mg of microorganisms/ml of saline. The suspension is dispensed into 5 ml vials, and then autoclaved for 10 minutes at 10 psi (69 kPa) to kill the microorganisms. After cooling, the therapeutic agent thus produced is stored at 4°C before use. A single dose consists of 0.1 ml of the suspension, which should be shaken vigorously immediately before use, containing 1 mg wet weight of M. vaccae. The dose is given by intradermal injection normally over the left deltoid muscle.

Of 148 fully treated leprosy patients, 79 were given M. vaccae therapy and 69 received a placebo. In the group receiving M. vaccae therapy, 17 showed symptoms of uveitis and of these, 13 were cleared of uveitis one year after therapy. In contrast, of the 69 patients receiving placebo, 12 showed symptoms of uveitis at the start of treatment and the uveitis cleared in only 4. This result is significant at p<0.005.

## Claims

1. Use of antigenic and/or immunoregulatory material derived from Mycobacterium vaccae in the manufacture of a therapeutic agent for the treatment of uveitis.

2. The use according to claim 1, wherein the antigenic and/or immunoregulatory material derived from M. vaccae comprises dead cells of M. vaccae.

3. The use according to claim 2, wherein the cells of M. vaccae have been killed by autoclaving.

4. The use according to claim 1, wherein the antigenic and/or immunoregulatory material derived from M. vaccae comprises the 65 kDa heat shock protein.

5. The use according to any one of the preceding claims, wherein the material derived from M. vaccae is derived from the strain as deposited at the National Collection of Type Cultures (NCTC) Central Public Health Laboratory, Colindale Avenue, London NW9 5HT, United Kingdom on February 13th, 1984 under the number NCTC 11659.

6. The use according to any one of the preceding claims, wherein the therapeutic agent contains, per dose, antigenic and/or immunoregulatory material from 10⁷ to 10¹⁰ M. vaccae microorganisms.

## Patentansprüche

1. Verwendung von antigenem und/oder immunregulierendem von Mycobacterium vaccae stammenden Material zur Herstellung eines Medikaments zur Behandlung von Uveitis.

2. Verwendung gemäß Anspruch 1, wobei das antigene und/oder immunregulierende von M. vaccae stammende Material tote Zellen von M. vaccae umfaßt.

3. Verwendung gemäß Anspruch 2, wobei die Zellen von M. vaccae durch Sterilisieren getötet wurden.

4. Verwendung gemäß Anspruch 1, wobei das antigene und/oder immunregulierende von M. vaccae stammende Material ein 65 kDa Hitzeschock-Protein enthält.

5. Verwendung gemäß einem der vorhergehenden Ansprüche, wobei das von M. vaccae stammende Material von dem beim National Collection of Type Cultures (NCTC) Central Public Health Laboratory, Colindale Avenue, London NW9 5HT, Vereinigtes Königreich, am 13. Februar 1984 unter der Nummer NCTC 11659 hinterlegtem Stamm stammt.

6. Verwendung gemäß einem der vorhergehenden Ansprüche, wobei das Medikament pro Dosis antigenes und/oder immunregulierendes Material von 10⁷ bis 10¹⁰ M. vaccae-Mikroorganismen enthält.

## Revendications

1. Utilisation d'une substance antigénique et/ou immunorégulatrice provenant de Mycobacterium vaccae pour la préparation d'un agent thérapeutique destiné au traitement de l'uvéite.

2. Utilisation selon la revendication 1, dans laquelle la substance antigénique et/ou immunorégulatrice provenant de M. vaccae comprend des cellules mortes de M. vaccae.

3. Utilisation selon la revendication 2, dans laquelle les cellules de M. vaccae ont été tuées par passage à l'autoclave.

4. Utilisation selon la revendication 1, dans laquelle la substance antigénique et/ou immunorégulatrice provenant de M. vaccae comprend la protéine du choc thermique de 65 kDa.

5. Utilisation selon l'une quelconque des revendications précédentes, dans laquelle la substance provenant de M. vaccae provient de la souche telle que déposée auprès de National Collection of Type Cultures (NCTC) Central Public Health Laboratory, Colindale Avenue, Londres NW9 5HT, Royaume-Uni, le 13 février 1984 sous le n° NCTC 11659.

6. Utilisation selon l'une quelconque des revendications précédentes, dans laquelle l'agent thérapeutique contient, par dose, la substance antigénique et/ou immunorégulatrice de 10⁷ à 10¹⁰ micro-organismes M. vaccae.
